Europäisches Patentamt

European Patent Office

Office européen des brevets

(11) Publication number: **0 102 457**
**B1**

# (12) EUROPEAN PATENT SPECIFICATION

(45) Date of publication of patent specification: **30.12.86**

(21) Application number: **83105530.6**

(22) Date of filing: **06.06.83**

(51) Int. Cl.⁴: **A 61 M 1/14, C 12 Q 1/00**

(54) **Device for measuring of concentration.**

(30) Priority: **15.06.82 SE 8203696**

(43) Date of publication of application:
**14.03.84 Bulletin 84/11**

(45) Publication of the grant of the patent:
**30.12.86 Bulletin 86/52**

(84) Designated Contracting States:
**AT BE CH DE FR GB IT LI LU NL**

(56) References cited:
**DE-A-2 450 612**
**DE-A-2 853 897**
**US-A-3 626 938**
**US-A-4 298 026**

(73) Proprietor: **Gambro Lundia AB**
**Box 10101**
**S-220 10 Lund (SE)**

(72) Inventor: **Claren, Jan Seivert**
**Protokollgränden 38**
**S-222 47 Lund (SE)**
Inventor: **Olsson, Lars-Göran**
**Docentgatan 6**
**S-223 63 Lund (SE)**

(74) Representative: **Boberg, Nils Gunnar Erik et al**
**Gambro AB Patent Department Box 10101**
**S-220 10 Lund (SE)**

Courier Press, Leamington Spa, England.

## Description

### Technical Field

This invention relates to a device for measuring of the concentration of a low molecular weight compound in a complex medium taken from a source of such a medium. The device comprises a dialyzer, possibly an enzyme reactor, and a measuring unit in connection with each other through a channel system, said measuring unit measures the concentration of the low molecular weight compound at the dialysate side of the dialyzer.

More specifically, the invention is relating to a device of the disclosed kind for measuring of glucose in blood of for example a patient.

### Technical Field

Devices of the disclosed kind, specifically for measuring of the concentration of glucose in blood are known through inter alia U.S. patent 4 229 542. Similar devices are described also in U.S. Patents Nos. 4 123 353, 4 266 021 and 4 298 026, and in British Patent 1 564 788. The measuring procedure with these known devices comprises an initial calibration step, in which the measuring unit is calibrated against a standard solution (calibration solution) containing the low molecular compound in a known concentration, whereafter the measuring of the low molecular weight compound in a complex medium is performed. For further information concerning this measuring procedure reference is made to any of the above-mentioned patents.

A drawback with this known measuring procedure is that calibration is performable only at the beginning of each measurement. This means that after an initial calibration one is left to rely upon this calibration result during the whole concentration measurement. This is of course insufficient, since the measuring unit (particularly if this is a measuring electrode) easily can give rise to systematic deviations during the measurement depending on for example temperature disturbances or the like. It would be desirable to be able to perform calibration also during the measuring procedure in order to achieve a check of the accuracy of the measuring unit and thereby a safer measuring result. If you, however, should try to perform calibration also during the measuring procedure using the devices according to the above-mentioned patents you will find that you will have to disconnect the source for the complex medium in order to use the same inlet instead for either feeding the calibration liquid to the device or feeding said liquid out of the device to a waste.

An object of the invention is therefore to meet this desire and to provide a device by means of which it is possible to calibrate the measuring unit also during the measuring procedure.

This object is obtained according to the invention by means of the device as defined in the following claims and which will be described in the following.

### Brief Description of the Invention

According to the invention there is provided a device for measuring of the concentration of a low molecular weight compound in a complex medium taken from a source of such a medium, which comprises a dialyzer, possibly an enzyme reactor, and a measuring unit in connection with each other through a channel system, said measuring unit measures the concentration of the low molecular weight compound at the dialysate side of the dialyzer. The device is characterized by comprising a valving means in a position upstream of the dialyzer which is adapted to alternatingly direct the complex medium and a calibration solution, respectively, through the other side of the dialyzer, whereby it is possible to calibrate the measuring unit during the concentration measurement without disconnecting the device from said source of the complex medium or the outlet for the consumed dialysis liquid and calibration solution.

As regards suitable and preferred embodiments of the valving means reference is made to the accompanying claims 2—11 and to the following description.

### Brief Description of the Drawings

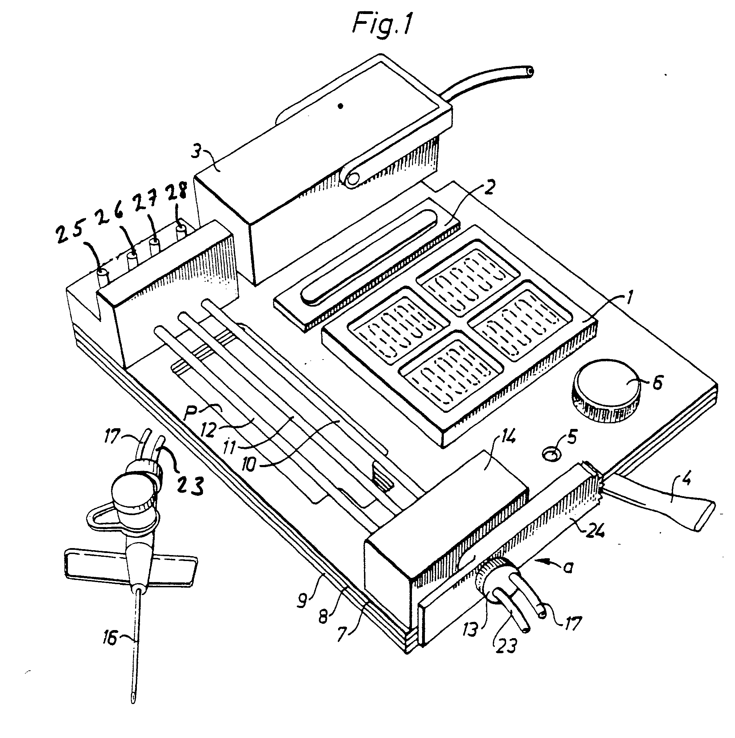

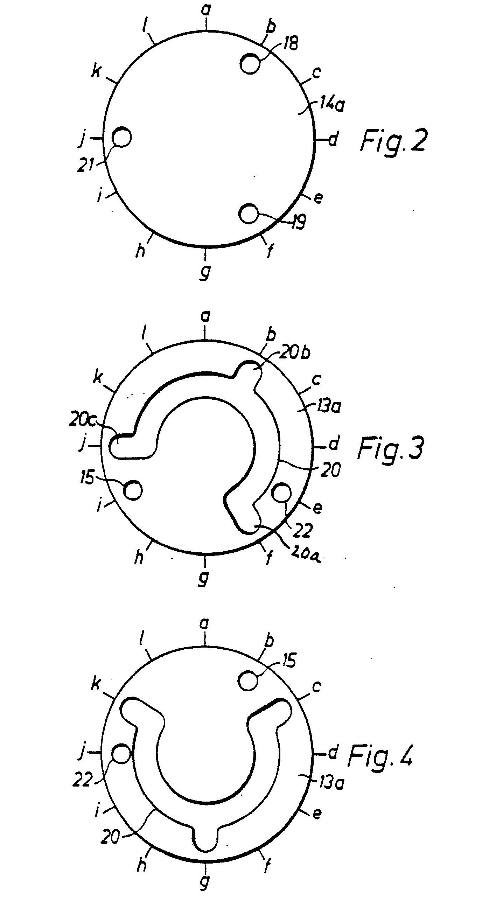

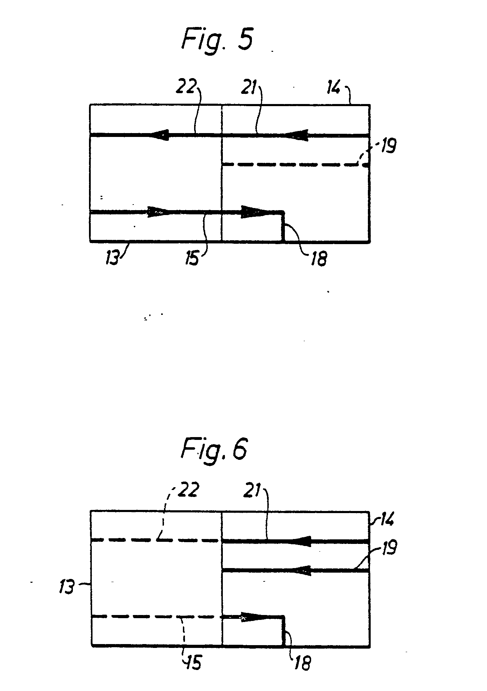

Fig. 1 shows a preferred embodiment of the present device, and Figs. 2—6 illustrate the function of a particularly preferred embodiment of the valving means of the device according to Fig. 1.

### Detailed Description of the Drawings

As is shown in Fig. 1 the device according to the present invention comprises preferably a dialyzer 1, an enzyme reactor 2, a measuring unit 3, a temperature sensor 4, a deaerator 5, and a flow changing device 6. These elements are known to the skilled person and will therefore not necessarily be described in more detail herein. For further information reference is instead made to any of the above-mentioned patents.

As is shown in Fig. 1, said elements are preferably arranged on the top side of a stack of plates 7, 8, 9 and in communication with each other through a channel system. Example of the preferred channel system may be the channel system as described in our EP—A—0 102 458 published on 14.3.84. Briefly, this channel system is composed of channels being formed between the stacked plates and a part of which is shown in Fig. 1 in the form of tubes 10, 11, 12 which are arranged over a pump hole P through the plates to thereby provide a means for pumping the individual liquids in the channel system.

From Fig. 1 it can be seen that the present device also includes a valving means (designated 1 in Fig. 1) which will be described in more detail in the following with reference to Figs. 2—6.

The valving means comprises preferably a valve piece 13, which is movable between two positions, and a stationary of fixed part 14, whereby the movable valve part 13 with its one end 13a conveniently is releasably attached to a corresponding end 14a of the fixed valve part 14.

The movable valve part 13 comprises an openable and closable inlet channel 15 for the complex medium, for example blood, which is adapted to be withdrawn from a patient by means of a cannula 16 and tube 17. The inlet channel 15 is in turn connectable to the dialyzer 1 via a corresponding outlet channel 18 in the fixed valve part 14 and the not shown channel system between the plates 10, 11, 12.

The fixed valve part 14 comprises an inlet channel 19 for calibration solution, whereby the inlet channel 19 is directly connectable to the outlet channel 18 via a groove 20 formed in the end 13a of the movable valve part 13, as will be described in the following.

Furthermore, the fixed valve part 14 preferably comprises an inlet channel 21 for anticoagulant, whereby this channel is directly connectable to the outlet channel 18 via said groove 20 of the movable valve part 13, and indirectly connectable to the outlet channel 18 via a corresponding channel 22 of the movable valve part 13 and a tube 23 which via a cannula 16 is in communication with the tube 17 for the complex medium and consequently, the inlet channel 15 in the movable valve part 13, as mentioned above.

The respective source for calibration solution and anticoagulant may for example be the container which is described in the Swedish Patent Application No. 8104146-7 Published on 4.1.83, and which can be in communication with the channel system in the plates 7, 8, 9 in the manner as described in our EP—A—0 102 458. Preferably, this container is provided with a level marking of the kind as described in the Swedish Patent Application No. 8104147-7, published 4.1.83.

In Fig. 1 you may furthermore see an inlet 25 for the calibration solution connected to the channel 19 for said solution, an inlet 26 for the anticoagulant connected to the inlet channel 21 for said anticoagulant, an inlet 27 for the dialysis liquid which is being pumped to the dialyzer by means of the pumping segment 10 and an outlet 28 for the consumed dialysis liquid, calibration solution and complex medium.

Operation of the Device
A. Calibration
When calibration is to be performed with the device according to the invention the valving means is set, preferably by means of an arm 24, in the position as shown in Figs. 2 and 3. In order to facilitate the comparison between the Figs. 2, 3 and 4 they have been provided with the letters a-l at fixed positions corresponding to the hour figures of a watch. As is shown in these figures the inlet channel 19 for calibration solution in the fixed valve part 14 is in communication with the groove 20 at 20a and via this groove at 20b in communication with the outlet channel 18 in the fixed valve part 14 which, as described, is in communication with the dialyzer 1 via the channel system between the plates 7, 8, 9. The inlet channel 21 for anticoagulant in the fixed valve part 14 is in communication with the groove 20 at 20c and via this groove at 20b in communication with the outlet channel 18. Furthermore, it can be seen from Figs. 2 and 3 that the communication between the inlet channel 15 for the complex medium in the movable valve part 13 and the outlet channel 18 is interrupted. The communication between the inlet channel 21 for anticoagulant in the fixed valve part 14 and the corresponding channel 22 in the movable valve part 13 is also interrupted.

During calibration calibration solution will thus be pumped from a not shown source in communication with an inlet 25 and through the channel system between the plates 7, 8, 9 into the inlet channel 19 in the fixed valve part 14 and via the groove 20a, 20, 20b in the movable valve part 13 into the outlet channel 18 in the fixed valve part 14 and from this via the channel system between the plates 7, 8, 9 to the dialyzer 1. From the dialyzer the calibration solution is then pumped via the channel system to a not shown waste container in communication with an outlet 28 in the manner as described in our PE—A—0 102 458. Simultaneously anticoagulant is pumped from a not shown source in communication with an inlet 26 through the tube 12 into the inlet channel 21 in the fixed valve part 14 and from this channel to the outlet 18 via the groove 20c, 20, 20b and from the outlet channel 18 through the same channel system as the calibration solution. Thereby mixing of anticoagulant and calibration solution is obtained when these two flows meet in the groove 20 and 20b.

B. Concentration Measurement
During concentration measurement of the low molecular weight compound (for example glucose) in the complex medium (for example blood) the valving means is set in the position as shown in Figs. 2 and 3, and 4, respectively. In this position the inlet channel 15 for the blood in the movable valve part 13 is in direct communication with the outlet channel 18 in the fixed valve part 14 and, as described above, in communication with the dialyzer 1 via the not shown channel system between the plates 7, 8, 9. Furthermore, in this position the inlet channel 21 for anticoagulant in the fixed valve part 14 is in communication with the outlet channel 18 via the corresponding channel 22 in the movable valve part 13, the tube 23, the cannula 16, the tube 17 and the inlet channel 15 in the movable valve part 13 for the blood. In this position the direct communication between the inlet channel 21 in the fixed valve part 14 and the outlet channel 18 is interrupted as well as the inlet channel 19 for calibration solution in the fixed valve part 14 and the outlet channel 18.

During concentration measurement of the low molecular weight compound the blood is thus withdrawn from for example a patient through the cannula 16 and pumped to the dialyzer 1 via the tube 17, inlet channel 15 in the movable valve part 13, the outlet channel 18 in the fixed valve part 14 and the not shown channel system between the plates 7, 8, 9. Simultaneously, anticoagulant is pumped into the inlet channel 21 in the fixed valve part 14 in the manner as described above and to the cannula 16 via the corresponding channel 22 in the movable valve part 13 and the tube 23. From the cannula 16 the anticoagulant will then follow the same flow pathway as the blood. Mixing of anticoagulant and blood within the cannula 16 is thereby provided where these two flows meet.

From the above description it is apparent that by means of the present device it is possible at intervals during the measurement of the low molecular weight compound in the complex medium to calibrate the measuring unit 3 by simply setting the valving means in its two respective positions for calibration and measuring.

The risk of coagulation in the cannula 16 and tube 17 during the calibration, i.e. when these two elements are filled with for example blood which then is quiescent, is avoided by the fact that the blood simultaneously and continuously is mixed with anticoagulant which thus meets the blood flow within the cannula 16.

A further essential advantage which is achieved according to the invention is that the tube 23 not necessarily must be disconnected from the cannular 16 during the calibration, which is particularly accentuated in cases where particularly long cannulas are used. According to the invention it is thus possible to maintain the tube 23 for anticoagulant in communication with the cannula 16 during the whole measuring procedure.

Industrial Applicability

The device according to the present invention is adapted for use in connection with concentration measurements of low molecular weight compounds in a complex medium. Specifically, it is adapted for measuring of concentration of glucose in blood of a patient, but it can also be used in quantitative and/or qualitative determination of low molecular weight compounds in other complex media than blood, for example microbiological culturing chambers.

**Claims**

1. A device for measuring of the concentration of a low molecular weight compound in a complex medium taken from a source of such a medium, which comprises a dialyzer (1), possibly an enzyme reactor (2), and a measuring unit (3) in connection with each other through a channel system, said measuring unit measures the concentration of the low molecular weight compound at the dialysate side of the dialyzer, characterized in that is comprises a valving means (a) in a position upstream of the dialyzer (1) which is adapted to alternatingly direct the complex medium and a calibration solution, respectively, through the other side of the dialyzer (1), whereby a calibration of the measuring unit is achievable at intervals during the measurement without disconnecting the device from said source of the complex medium or the outlet for the consumed dialysis liquid and calibration solution.

2. A device according to claim 1, characterized in that the valveing means comprises an openable and closable inlet channel (15) for the complex medium, an openable and closable inlet channel (19) for calibration solution and a common outlet channel (18) for the complex medium and calibration solution.

3. A device according to claim 2, characterized in that the valve means comprises a part (13), which is movable between two positions, and a fixed part (14).

4. A device according to claim 3, characterized in that the openable and closable inlet channel (15) for the complex medium is arranged in the movable valve part (13), while the openable and closable inlet channel (19) for calibration solution and the common outlet channel (18) are arranged in the fixed valve part (14).

5. A device according to any of claims 2—4, characterized in that the valving means furthermore comprises an inlet channel (21) for an anticoagulant, which inlet channel is directly and indirectly connectable to the common outlet channel (18).

6. A device according to claim 5, characterized in that the inlet channel (21) for anticoagulant is arranged in a fixed valve part (14).

7. A device according to claim 5 or 6, characterized in that the inlet channel (21) for anticoagulant is indirectly connectable to the common outlet channel (18) via an openable and closable channel (22) in the movable valve part (13) in communication with the inlet channel (15) for the complex medium.

8. A device according to any of claims 3—7, characterized in that one end (13a) of the movable valve part (13) is releasably attached to a corresponding end (14a) of the fixed valve part (14).

9. A device according to claim 8, characterized in that the inlet channel (21) for anticoagulant is directly connectable to the common outlet channel (18) via a groove (20) in said one end (13a) of the movable valve part (13).

10. A device according to claims 3 and 5, characterized in that the outlet channel (21) for anticoagulant is in communication with the outlet channel (18) as well during the measurement as during the calibration.

11. A device according to claim 9, characterized in that the inlet channel (21) for anticoagulant in the fixed valve part (14) and the inlet channel (15) for the complex medium in the movable valve part (13) are simultaneously in communication with the common outlet channel (18), when the movable valve part (13) is in its one position, and that the inlet channel (21) for anticoagulant and inlet channel (19) for calibration solution are simultaneously in communication with the common outlet channel (18) via said groove (20) in the movable valve part (13), when the movable valve part is in its second position, whereby the communication between the inlet channel (15) for the complex medium and the outlet channel (18) is interrupted.

12. A device according to any of claims 3—11, characterized in that the movable valve part (13) is movable between its two positions by means of an arm (24) which is provided on the movable valve part.

## Patentansprüche

1. Vorrichtung zum Messen der Konzentration einer Verbindung mit niedrigem Molekulargewicht in einem komplexem Medium, das aus einer Quelle eines solchen Mediums genommen wird, die einen Dialysator (1), möglicherweise einen Enzymreaktor (2), und eine Meßeinheit (3) in Verbindung miteinander über ein Kanalsystem aufweist, wobei die Meßeinheit die Konzentration der Verbindung mit niedrigem Molekulargewicht an der Dialysatseite des Dialysators mißt, dadurch gekennzeichnet, daß sie ein Ventilmittel (a) in einer Position aufstromig von dem Dialysator (1) aufweist, das in geeigneter Weise abwechselnd das komplexe Medium bzw. eine Kalibrierlösung durch die andere Seite des Dialysators (1) führt, wodurch ein Kalibrieren der Meßeinheit in Intervallen während des Messens erreichbar ist, ohne die Vorrichtung von der Quelle des komplexen Mediums oder dem Auslaß für verbrauchte Dialyseflüssigkeit und Kalibrierlösung abzutrennen.

2. Vorrichtung nach Anspruch 1, dadurch gekennzeichnet, daß das Ventilmittel einen öffnungsfähigen und schließbaren Einlaßkanal (15) für das komplexe Medium, einen öffnungsfähigen und schließbaren Einlaßkanal (19) für Kalibrierlösung und einen gemeinsamen Auslaßkanal (18) für das komplexe Medium und Kalibrierlösung aufweist.

3. Vorrichtung nach Anspruch 2, dadurch gekennzeichnet, daß das Ventilmittel ein Teil (13), welches zwischen zwei Stellungen bewegbar ist, und ein festes Teil (14) aufweist.

4. Vorrichtung nach Anspruch 3, dadurch gekennzeichnet, daß der öffnungsfähige und schließbare Einlaßkanal (15) für das komplexe Medium in dem bewegbaren Ventilteil (13) angeordnet ist, während der öffnungsfähige und schließbare Einlaßkanal (19) für die Kalibrierlösung und der gemeinsame Auslaßkanal (18) in dem festen Ventilteil (14) angeordnet sind.

5. Vorrichtung nach einem der Ansprüche 2 bis 4, dadurch gekennzeichnet, daß das Ventilmittel ferner einen Einlaßkanal (21) für ein Antikoagulans aufweist, wobei der Einlaßkanal direkt und indirekt mit dem gemeinsamen Auslaßkanal (18) verbindbar ist.

6. Vorrichtung nach Anspruch 5, dadurch gekennzeichnet, daß der Einlaßkanal (21) für Antikoagulans in einem festen Ventilteil (14) angeordnet ist.

7. Vorrichtung nach Anspruch 5 oder 6, dadurch gekennzeichnet, daß der Einlaßkanal (21) für Antikoagulans indirekt mit dem gemeinsamen Auslaßkanal (18) über einen öffnungsfähigen und schließbaren Kanal (22) in dem bewegbaren Ventilteil (13) in Verbindung mit dem Einlaßkanal (15) für das komplexe Medium verbindbar ist.

8. Vorrichtung nach einem der Ansprüche 3 bis 7, dadurch gekennzeichnet, daß eine Ende (13a) des bewegbaren Ventilteils (13) lösbar an einem entsprechenden Ende (14a) des festen Ventilteils (14) angebracht ist.

9. Vorrichtung nach Anspruch 8, dadurch gekennzeichnet, daß der Einlaßkanal (21) für Antikoagulans direkt mit dem gemeinsamen Auslaßkanal (18) über eine Nut (20) in dem einen Ende (13a) des bewegbaren Ventilteils (13) verbindbar ist.

10. Vorrichtung nach Ansprüchen 3 und 5, dadurch gekennzeichnet, daß der Auslaßkanal (21) für Antikoagulans mit dem Auslaßkanal (18) sowohl während des Messens als auch während des Kalibrierens in Verbindung steht.

11. Vorrichtung nach Anspruch 9, dadurch gekennzeichnet, daß der Einlaßkanal (21) für Antikoagulans in dem festen Ventilteil (14) und der Einlaßkanal (15) für das komplexe Medium in dem bewegbaren Ventilteil (13) gleichzeitig mit dem gemeinsamen Auslaßkanal (18) in Verbindung stehen, wenn sich das bewegliche Ventilteil (13) in seiner einen Stellung befindet, und daß der Einlaßkanal (21) für Antikoagulans und der Einlaßkanal (19) für Kalibrierlösung mit dem gemeinsamen Auslaßkanal (18) über die Nut (20) in dem beweglichen Ventilteil (13) gleichzeitig in Verbindung stehen, wenn sich das bewegliche Ventilteil in seiner zweiten Stellung befindet, wodurch die Verbindung zwischen dem Einlaßkanal (15) für das komplexe Medium und dem Auslaßkanal (18) unterbrochen ist.

12. Vorrichtung nach einem der Ansprüche 3 bis 11, dadurch gekennzeichnet, daß das bewegliche Ventilteil (13) zwischen seinen zwei Stellungen mittels eines Armes (24) bewegbar ist, der auf dem beweglichen Ventilteil vorgesehen ist.

## Revendications

1. Appareil pour mesurer la concentration d'un composé de bas poids moléculaire dans un milieu complexe prélevé d'une source d'un tel milieu, qui comprend un dialyseur (1), éventuellement un réacteur enzymatique (2) et un dispositif de mesure (3) relié au précédent par un système de canalisations, ledit dispositif de mesure mesurant la concentration d'un composé de bas poids moléculaire du côté dialysat du dialyseur, caractérisé en ce qu'il comprend un système de valve (a) disposé en amont du dialyseur (1) pour diriger alternativement le milieu complexe et une solution d'étalonnage, respectivement, vers l'autre côté du dialyseur (1) de façon à permettre un étalonnage du dispositif de mesure à intervalles pendant l'opération de mesure sans débrancher l'appareil de ladite source de milieu complexe ou de la sortie pour le liquide de dialyse usé et la solution d'étalonnage.

2. Appareil suivant la revendication 1, caractérisé en ce que le système de valve comprend un passage d'entrée ouvrable et fermable (15) pour le milieu complexe, un passage d'entrée ouvrable et fermable (19) pour la solution d'étalonnage et un passage commun de sortie (18) pour le milieu complexe et la solution d'étalonnage.

3. Appareil suivant la revendication 2, caractérisé en ce que le système de valve comprend un élément (13) mobile entre deux positions, et une pièce fixe (14).

4. Appareil suivant la revendication 3, caractérisé en ce que le passage d'entrée ouvrable et fermable (15) pour le milieu complexe est ménagé dans l'élément mobile de valve (13), tandis que le passage d'entrée ouvrable et fermable (19) pour la solution d'étalonnage, ainsi que le passage de sortie commun (18) sont ménagés dans la pièce fixe de valve (14).

5. Appareil suivant l'une quelconque des revendications 2 à 4, caractérisé en ce que le système de valve comprend en outre un passage d'entrée (21) pour anticoagulant, ledit passage d'entrée étant reliable directement et indirectement au passage commun de sortie (18).

6. Appareil suivant la revendication 5, caractérisé en ce que le passage d'entrée (21) pour anticoagulant est ménagé dans la pièce fixe de valve (14).

7. Appareil suivant la revendication 5 ou la revendication 6, caractérisé en ce que le passage d'entrée (21) pour anticoagulant peut être relié indirectement au passage commun de sortie (18) par un passage ouvrable et fermable (22) dans l'élément mobile de valve (18) qui communique avec le passage d'entrée (15) pour le milieu complexe.

8. Appareil suivant l'une quelconque des revendications 3 à 7, caractérisé en ce que l'une des extrémités (13a) de l'élément mobile de valve (13) est fixée de façon détachable à une extrémité correspondante (14a) de la pièce fixe de valve (14).

9. Appareil suivant la revendication 8, caractérisé en ce que le passage d'entrée (21) pour anticoagulant peut être relié directement au passage commun de sortie (18) par une rainure (20) ménagée dans l'extrémité précitée (13a) de l'élément mobile de valve (13).

10. Appareil suivant les revendications 3 et 5, caractérisé en ce que le passage de sortie (21) pour l'anticoagulant communique avec le passage de sortie (18) aussi bien pendant l'opération de mesure que pendant l'opération d'étalonnage.

11. Appareil suivant la revendication 9, caractérisé en ce que le passage d'entrée (21) pour anticoagulant ménagé dans la pièce fixe de valve (14) et le passage d'entrée (15) pour le milieu complexe dans l'élément mobile de valve (13) sont simultanément en communication avec le passage commun de sortie (18) lorsque l'élément mobile de valve (13) est dans sa première position, et en ce que le passage d'entrée (21) pour l'anticoagulant et le passage d'entrée (19) pour la solution d'étalonnage sont simultanément en communication avec le passage commun de sortie (18) par l'intermédiaire de la rainure (20) ménagée dans l'élément mobile de valve (13) lorsque l'élément mobile de valve est dans sa deuxième position, de sortie que la communication entre le passage d'entrée (15) pour le milieu complexe et le passage de sortie (18) est interrompue.

12. Appareil suivant l'une quelconque des revendications 3 à 11, caractérisé en ce que l'élément mobile de valve (13) peut être déplacé entre ses deux positions par un bras (24) qui est monté sur l'élément mobile de valve.

# Fig.1

Fig.2

Fig.3

Fig.4

## Fig. 5

## Fig. 6